## Europäisches Patentamt

(19) **European Patent Office**

**Office européen des brevets**

(11) Numéro de publication: **0 223 809**

**B1**

(12) **FASCICULE DE BREVET EUROPEEN**

(45) Date de publication du fascicule du brevet: **07.11.90**

(51) Int. Cl.⁵: **C 12 M 1/18**

(21) Numéro de dépôt: **86903425.6**

(22) Date de dépôt: **06.06.86**

(86) Numéro de dépôt international:
**PCT/FR86/00195**

(87) Numéro de publication internationale:
**WO 86/07376 18.12.86 Gazette 86/27**

(54) **PROCEDE DE PRODUCTION DE SPORES DE CHAMPIGNONS FILAMENTEUX.**

(30) Priorité: **06.06.85 FR 8508555**

(43) Date de publication de la demande:
**03.06.87 Bulletin 87/23**

(45) Mention de la délivrance du brevet:
**07.11.90 Bulletin 90/45**

(84) Etats contractants désignés:
**AT BE CH DE FR GB IT LI LU NL SE**

(56) Documents cités:
**FR-A- 373 828**
**FR-A-2 044 588**
**FR-A-2 486 097**
**GB-A-1 581 832**

(73) Titulaire: **INSTITUT FRANCAIS DE RECHERCHE SCIENTIFIQUE POUR LE DEVELOPPEMENT EN COOPERATION (ORSTOM)**
**24, rue Bayard**
**F-75008 Paris (FR)**

(72) Inventeur: **RAIMBAULT, Maurice**
**6, hameau de la Frégate Port Sud**
**F-91650 Breuillet (FR)**
Inventeur: **ROUSSOS, Sevastianos**
**Callé Homero 1804 N 404**
**Colonia Los Morales 11510 Mexico DF (MX)**

(74) Mandataire: **Ahner, Francis et al**
**CABINET REGIMBEAU, 26, avenue Kléber**
**F-75116 Paris (FR)**

# EP 0 223 809 B1

**Description**

La présente invention concerne un procédé de production de spores de champignons filamenteux.

L'utilisation des champignons filamenteux est très répandue dans des domaines aussie variés que les fermentations alimentaires, l'industrie pharmaceutique et la production d'enzymes ou de molécules biologiques par biosynthèse ou hémisynthèse.

Les spores de champignons filamenteux sont la forme de résistance et de reproduction de ces microorganismes. Ces spores constituent le point de départ de toutes ces applications puisqu'elles peuvent servir de forme de conservation et de lancement de l'opération, mais également intervenir de façon massive dans le processus lui-même soit en tant qu'inoculum ou en tant que spores elles-mêmes pour réaliser les transformations de bio-conversion attendues.

Différentes techniques sont connues pour la production de ces spores. La plus ancienne, mais la plus artisanale consiste à cultiver l'organisme à la surface d'un milieu gélosé soit en boîte de Petri au laboratoire, soit en fioles de Roux pour les applications pratiques. Cette technique quoique sûre, puisqu'une aseptie complète peut être réalisée, pose de graves problèmes de récolte des spores et de manipulation d'un nombre important de fioles lorsque la quantité désirée s'élève quelque peu. C'est la raison pour laquelle une telle pratique ne peut rester qu'artisanale et ne trouver d'application que dans des cas particuliers.

La technique de production de spores en plateaux fait appel à la culture du champignon sur des substrats végétaux tels que son de blé, paille et divers produits ou résidus amylacés disposés en couche de quelques centimètres d'épaisseur et placées dans des étuves d'incubation. Des dispositifs automatiques complexes de chargement et déchargement des plateaux ont été proposés. Toutefois, il reste que la produit obtenu est constitué non pas de spores pures, mais d'un mélange de spores, de mycelium du champignon et des résidus végétaux. D'autre part, le maintien des conditions aseptiques est très délicat. La récolte des spores, la contamination ambiante et la variabilité de l'organisme sont des inconvénients majeurs.

Plus récemment on a montré la possibilité de produire des spores de champignons dans des cultures liquides en utilisant des fermenteurs stérilisables. Il s'agit sans doute d'un progrès réel, mais cela n'est possible que dans des conditions particulières et avec un nombre limité de champignons. D'autre part, la suspension recueille contient non seulement les spores, mais également une quantité importante de métabolites et tous les débris cellulaires qui peuvent être gênants. Enfin, le maintien des conditions d'aération efficace du milieu liquide pendant de longues périodes d'incubation entraînent des dépenses d'énergie coûteuses.

Par ailleurs, parmi les nombreux dispositifs de fermenteur qui ont été proposés pour la culture des microorganismes en milieu liquide, on trouve des fermenteurs à disques rotatifs, dont le principe d'utilisation est basé essentiellement sur la rotation permanente des disques tout au long de l'incubation et qui plongent alternativement dans le milieu nutritif liquide et dans l'atmosphère.

La présente invention concerne un procédé de production de spores de champignons filamenteux, caractérisé en ce qu'il implique la mise en oeuvre, dans un fermenteur à disques rotatifs, des opérations successives suivantes:

a) on ajoute un agent de solidification dans un milieu de culture contenant au moins un substrat de croissance ainsi que des agents de culture;

b) on dispose ce milieu de culture sur les disques rotatifs dans le fermenteur;

c) on stérilise l'ensemble du fermenteur;

d) on refroidit le milieu de culture à une température restant supérieure au point de solidification dudit milieu;

e) on inocule ledit milieu de culture avec des spores de champignons filamenteux;

f) on homogénéise ledit milieu de culture par rotation lente des disques du fermenteur;

g) tout en maintenant une rotation lente des disques du fermenteur, on solidifie le milieu absorbé sur les disques rotatifs par abaissement brusque de la température du fermenteur;

h) après immobilisation des disques rotatifs, on laisse incuber le milieu inoculé en assurant dans le fermenteur une circulation d'air stérile, d'humidité et de température contrôlées, pendant le temps nécessaire à la maturation des conidies, et

i) après développement uniforme des spores à la surface des disques du fermenteur, on sépare et on récolte les spores par balayage des disques animés d'un mouvement de rotation rapide, à l'aide d'un fluide contenant de préférence un agent tensio-actif et/ou des billes calibrées, la biomasse mycélienne restant emprisonnée dans le milieu de culture solidifié qui reste fixé sur les disques du fermenteur.

La présente invention concerne aussi le dispositif nécessaire à la mise en oeuvre du procédé décrit ci-dessus; il s'agit d'un fermenteur à disques rotatifs, pourvu de systèmes de régulation de la température et de l'humidité, et d'un moteur permettant la mise en rotation de l'axe supportant e'empilement des disques.

D'autres caractéristiques et avantages de la présente invention apparaîtront à la lecture de la description détaillée faite ci-après, notamment en regard de la figure annexée qui illustre un mode de réalisation particulier du dispositif nécessaire à la mise en oeuvre du procédé de l'invention.

Ce nouveau procédé pour la production de spores de champignons filamenteux allie l'avantage de la culture de surface sur un milieu solidifé, qui reste la technique la plus sûre et la plus générale de sporulation, avec l'utilisation d'un fermenteur à disques rotatifs particulier dont le principe d'utilisation a été fondamentalement modifié de façon à obtenir une grande surface de sporulation et permettant en particulier de récolter aisément uniquement les spores par simple lavage des surfaces, la biomasse mycélienne restant emprisonnée dans le milieu solidifié. Toutes les opérations peuvent être réalisées dans le même appareil stérilisable, assurant une grande simplicité et une aseptie stricte.

Le principe de ce procédé est basé sur le fait que le milieu de culture nutritif contenant le ou les substrats de croissance et les agents de cultures est additionné d'un agent de solidification, par exemple de la gélose. Après stérilisation, le milieu est refroidi et sa température est maintenue au-dessus du point de solidification, par exemple 50°C. L'inoculum de spores est alors ajouté et une rotation lente des disques permet d'homogénéiser le milieu.

Tout en maintenant une lente rotation des disques, l'atmosphère est brutalement refroidie de façon à permettre la solidification du milieu à la surface des disques.

Lorsque le milieu de culture est réparti sur les disques, la rotation est stoppée et la température du dispositif est amenée à la température d'incubation. Un flux d'air stérile, d'humidité et de température contrôlés est maintenu pendant toute la période d'incubation de façon à maintenir les conditions favorable de développement du champignon. Notons que pendant cette incubation aucune agitation mécanique n'est nécessaire.

L'organisme se développe ainsi uniformément dans toute la masse du milieu solidifié. Après 1 à 2 jours les surfaces libres sont recouvertes d'un tapis homogène de filaments aériens. Suivant l'organisme utilisé la sporulation se produit après 2 à 3 jours d'une façon très régulière et synchrone sur toutes les surfaces disponibles. Les spores contenues dans les têtes conidiennes aériennes se trouvent ainsi à l'extérieur des disques (5), alors que le mycelium végétatif reste emprisonné dans le milieu solidifié (6). L'incubation peut être poursuivie jusqu'à 7 à 10 jours pour permettre la maturation des têtes conidiennes.

La récolte des spores peut alors être faite de façon particulièrement aisée en introduisant dans le fermenteur un volume d'eau stérile additionné d'un agent tensio-actif, par exemple du TWEEN 80® et en maintenant une rotation rapide des disques pendant 10 minutes environ.

Ceci permet un lavage très efficace des surfaces des disques provoquant la mise en suspension des spores à l'exclusion de la biomasse mycélienne et de la majorité des métabolites qui sont fortement fixées à l'intérieur du milieu gélosé. Plusieurs lavages successifs peuvent être pratiqués. Les spores peuvent alors être centrifugées ou décantées, lavées, et séchées par évaporation sous vide, lyophilisation ou atomisation.

Selon un autre mode de récolte possible, on peut introduire des billes calibrées d'un diamètre inférieur à la distance interface des disques pour favoriser la dispersion des spores dans l'atmosphères. Les spores sont alors récoltées à sec grâce à la rotation des disques couplée à un balayage d'air stérile suffisamment élevé pour créer des turbulences et entraîner les spores qui sont alors récoltées grâce à un dispositif de filtration de l'air à la sortie du fermenteur.

Lorsque les spores ont été récoltées, le lavage du fermenteur est réalisé en introduisant un liquide détergent à chaud qui, couplé à une agitation rapide, provoque la fusion du milieu, la stérilisation de la biomasse résiduelle et le nettoyage du dispositif.

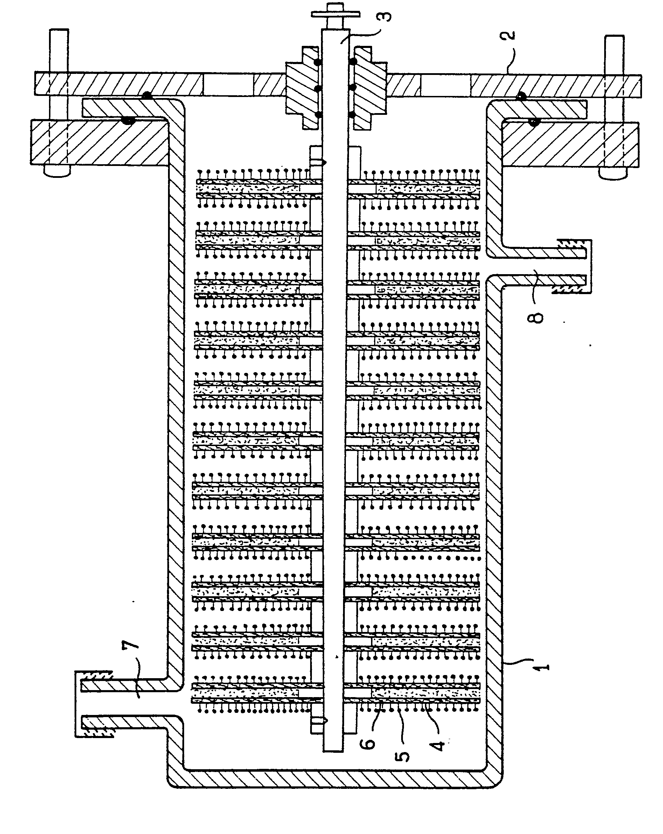

Selon un mode préféré de mise en oeuvre du procédé, le réacteur décrit sur la figure 1 est de forme cylindrique et réalisé en matériaux pouvant supporter la stérilisation. Le fermenteur est constitué d'une cuve cylindrique 1 munie à une extrémité d'une platine 2, munie d'un axe rotatif 3 supportant un empilement de disques rigides 4 d'un diamètre légèrement inférieur au diamètre interne de la cuve. La matière, l'épaisseur et la surface des disques peuvent être quelconques, pourvu qu'ils permettent une bonne rétention et une répartition homogène de milieu au cours de la rotation et de la phase de solidification. Ces disques 4 peuvent être constitués simplement par une ou deux grilles d'acier de quelques mm d'épaisseur et de maille de 2 à 5 mm. L'espacement et la disposition de ces grilles peuvent être variables. Dans la figure 1 décrivant un exemple de mise en oeuvre de ce dispositif, les disques sont constitués de deux grilles d'acier de 2 mm d'épaisseur espacées de 2 mm de telle sorte qu'après répartition et solidification du milieu, on obtienne un disque de 6 mm d'épaisseur. Dans un mode préféré de mise en oeuvre, l'espacement entre les disques gélosés est de 10 mm.

Un balayage forcé du réacteur par un flux d'air stérile humidifié par barbotage doit être prévu par une entrée haute (7) et une sortie basse (8) qui permettent également le remplissage et la vidange du réacteur. Des dispositifs de contrôle et de régulation de la température, de l'humidité relative et de la composition de l'atmosphère du réacteur peuvent se révéler utiles.

Le réacteur tel qu'il vient d'être décrit a été utilisé pour la mise en oeuvre de l'invention sous forme d'un réacteur de 1500 cm$^3$ muni de 10 plateaux de 5 mm représentant une surface libre de 1 270 cm$^2$.

Les essais ont porté sur la production de spores de champignons appartenant aux genres Aspergillus, Trichoderma, Penicillium. En l'occurrence une souche d'Aspergillus hennebergii du groupe A. niger et une souche de Penicillium isolée au laboratoire ont été choisies pour représenter les genres Aspergillus et Penicillium. Deux souches de collection internationale, Trichoderma harzianum CCM—F—470 et Penicillium camembertii CCM—F—378 ont été choisies pour représenter les genres Trichoderma et Penicillium.

Dans un exemple de mise en oeuvre préféré du procédé, le milieu de culture est composé pour un litre d'eau de 100 g de farine de manioc, de 4 g de $KH_2PO_4$, de 8 g de $(NH_4)_2SO_4$, de 2 g d'urée et de 20 g d'Agar. 300 ml de ce milieu sont introduits dans le réacteur qui est alors stérilisé à 120°C pendant 30 minutes. Le

3

dispositif est alors refroidi et lorsque la température du milieu est de 50°C l'inoculum de spores est introduit stérilement. Le milieu est alors homogénéisé par une rotation des disques à raison de 60 t/mn pendant quelques minutes. Après homogénéisation, un courant d'air stérile et froid à raison de 200 l/h permet la solidification du milieu de culture sur les grilles et la constitution des disques de milieu. Après 30 minutes, la rotation est stoppée et le fermenteur est aéré par un débit de 10 litres/h d'air stérile saturé d'eau à la température d'incubation, qui peut être de 30°C, mais varie avec l'organisme cultivé.

Après 7 jours, la récolte des spores est réalisée en introduisant dans le fermenteur 500 ml d'eau stérile contenant quelques gouttes de TWEEN 80. L'aération est stoppée et la rotation des disques à 100—200 t/mm est maintenue pendant 10 minutes. La suspension de spores est alors recueillie, et le nombre de spores est calculé par un comptage microscopique direct sur une cellule de Malassez après une dilution adéquate. Deux lavages supplémentaires utilisant 250 ml d'eau sont réalisés. La suspension de spores est alors décantée et, après élimination du surnageant, une poudre sèche de spores est obtenue par évaporation sous vide de la suspension concentrée. Cette poudre de spores permet une remise en suspension aisée des spores pour leur utilisation, sans phénomènes tensio-actifs gênants.

Le tableau I indique les résultats obtenus pour quatre souches de champignons. On constate que le premier lavage permet de récolter 75 à 90% des spores contenues dans le dispositif et d'obtenir des suspensions de spores concentrées contenant au moins $1,1 \times 10^8$ à $4,2 \times 10^8$ spores par ml.

La quantité de spores formée par cm² varie de $3 \times 10^7$ à $2,2 \times 10^8$ spores/cm². Le rendement pondéral en spores rapporté à l'a quantité de substrat introduite dans le fermenteur varie de 5 à 14%, ce qui représente un taux de conversion en spores important.

Compte tenu de ce qui précède, la présente invention permet de produire des spores de champignons en conditions aseptiques et selon une technique relativement simple, d'une mise en oeuvre aisée ne nécessitant pas de manipulations longues et côteuses. Elle permet également d'éviter tous risques de contamination de l'environnement. D'autre part, la présente invention est caractérisée par le fait que l'on obtient des spores pures sans contamination par d'autres microorganismes ni par des métabolites ou résidus mycéliens qui sont retenus essentiellement dans le dispositif lors de la récolte. Les rendements obtenus sont supérieurs ou comparables à ce que l'on peut obtenir par les autres techniques habituelles d'une mise en oeuvre délicate. Enfin cette invention est caractérisée par le faible encombrement des matériels en fonction des quantités de spores obtenues.

TABLEAU I

| | Aspergillus niger (henebergii) | Trichoderma harzianum | Penicillium sp | Penicillium camembertii |
|---|---|---|---|---|
| 1er lavage, 500 ml | 75 % | 76 % | 87 % | 92 % |
| 2ème lavage, 250 ml | 15 % | 24 % | 11 % | 6 % |
| 3ème lavage, 250 ml | 10 % | 5 % | 2 % | 2 % |
| Nombre de spores récoltées dans 1 l | $7,3 \times 10^{10}$ | $2,8 \times 10^{11}$ | $1,5 \times 10^{11}$ | $3,8 \times 10^{10}$ |
| Concentration en spores/ml   1è | $1,1 \times 10^{8}$ | $4,2 \times 10^{8}$ | $2,6 \times 10^{8}$ | $6,9 \times 10^{7}$ |
|   2è | $4,3 \times 10^{7}$ | $2,6 \times 10^{8}$ | $6,6 \times 10^{7}$ | $9,1 \times 10^{6}$ |
|   3è | $3,0 \times 10^{7}$ | $5,6 \times 10^{7}$ | $1,2 \times 10^{4}$ | $3,0 \times 10^{6}$ |
| Nombre de spores formées par cm2 | $5,75 \times 10^{7}$ | $2,2 \times 10^{8}$ | $1,2 \times 10^{8}$ | $3,0 \times 10^{7}$ |
| Nombre de spores par g de substrat | $2,5 \times 10^{9}$ | $9,3 \times 10^{9}$ | $5,0 \times 10^{9}$ | $1,25 \times 10^{9}$ |
| Poids de 1 spore (g) | $6,0 \times 10^{-11}$ | $1,3 \times 10^{-11}$ | $1,1 \times 10^{-11}$ | $4,0 \times 10^{-11}$ |
| Rendement pondéral global rapporté au substrat carboné, poids spores/g | 14,6 % | 12,1 % | 5,75 % | 5 % |

EP 0 223 809 B1

**Revendications**

1. Procédé de production de spores de champignons filamenteux, caractérisé en ce qu'il implique la mise en oeuvre, dans un fermenteur à disques rotatifs, des opérations successives suivantes:

a) on ajoute un agent de solidification dans un milieu de culture contenant au moins un substrat de croissance ainsi que des agents de culture;

b) on dispose ce milieu de culture sur les disques rotatifs dans le fermenteur;

c) on stérilise l'ensemble du fermenteur;

d) on refroidit le milieu de culture à une température restant supérieure au point de solidification dudit milieu;

e) on inocule ledit milieu de culture avec des spores de champignons filamenteux;

f) on homogénéise ledit milieu de culture par rotation lente des disques du fermenteur;

g) tout en maintenant une rotation lente des disques du fermenteur, on solidifie le milieu par abaissement brusque de la température du fermenteur, ce qui a pour effet de répartir le milieu sur les disques;

h) après immobilisation des disques rotatifs, on laisse incuber le milieu inoculé en assurant dans le fermenteur une circulation d'air, d'humidité et de température contrôlées, pendant le temps nécessaire à la maturation des conidies, et

i) après développement uniforme des spores à la surface des disques du fermenteur, on sépare et on récolte les spores par balayage des disques animés d'un mouvement de rotation rapide, à l'aide d'un fluide contenant de préférence un agent tensio-actif et/ou des billes calibrées, la biomasse mycélienne restant emprisonnée dans le milieu de culture solidifié qui reste fixé sur les disques du fermenteur.

2. Procédé selon la revendication 1, caractérisé en ce qu'il est appliqué à la production de spores de champignons dont la forme de reproduction est constituée par des conidies.

3. Procédé selon l'une des revendications 1 et 2, caractérisé en ce qu'il est appliqué à la production de spores de champignons appartenant aux genres Aspergillus, tel que Aspergillus niger, Trichoderma, tel que Trichoderma harzianum ou Penicillium, tel que Penicillium camembertii.

4. Procédé selon l'une des revendications 1 et 3, caractérisé en ce que les agents de culture sont constitués d'une source de carbone, telle que l'amidon, d'une source d'azote, telle qu'un mélange de sulfate d'ammonium et d'urée, d'une source de phosphore et d'autres composés minéraux ou organiques.

5. Procédé selon l'une des revendications 1 et 4, caractérisé en ce que l'agent de solidification ajouté au cours de l'étape a) est de la gélose.

6. Procédé selon l'une des revendications 1 et 5, caractérisé en ce que le fluide de lavage utilisé au cours de l'étape i) est de l'eau stérile, additionnée d'un agent tensio-actif tel que du TWEEN 80.

7. Procédé selon l'une des revendications 1 à 6, caractérisé en ce que les billes calibrées utilisées au cours de l'étape i) ont un diamètre inférieur à la distance interface des disques.

8. Procédé selon l'une des revendications 1 et 7, caractérisé en ce que la suspension des spores récoltées est concentrée par centrifugation ou décantation, lavée puis séchée par évaporation sous vide, lyophilisation ou atomisation.

9. Dispositif pour la mise en oeuvre du procédé selon l'une des revendications 1 à 8, caractérisé en ce qu'il se présente sous la forme d'un fermenteur à disques rotatifs, pourvu de systèmes de régulation de la température et de l'humidité, et d'un moteur permettant la mise en rotation de l'axe supportant l'empilement des disques, disques dont au moins une surface est constituée par une grille ayant une ouverture de maille de 2 à 5 mm.

**Patentansprüche**

1. Verfahren zur Gewinnung von Sporen von Fadenpilzen, dadurch gekennzeichnet, daß man in einem Fermenter mit sich drehenden Scheiben die folgenden Arbeitsgänge nacheinander durchführt:

a) einem Kulturmedium, das mindestens ein Wachstumssubstrat sowie Kulturagentien enthält, gibt man ein Verfestigungs-Agens zu;

b) man bringt dieses Kulturmedium auf die sich drehenden Scheiben in dem Fermenter auf;

c) man sterilisiert den gesamten Fermenter;

d) man kühlt das Kulturmedium auf eine Temperatur ab, die oberhalb des Erstarrungspunktes des Mediums bleibt;

e) man inokuliert dieses Kulturmedium mit Sporen von Fadenpilzen;

f) man homogenisiert das Kulturmedium durch langsames Drehen der Scheiben des Fermenters;

g) man läßt das Medium durch plötzliche Senkung der Temperatur des Fermenters erstarren unter Aufrechterhaltung einer langsamen Drehung der Scheiben des Fermenters, so daß sich das Medium auf den Scheiben verteilt;

h) nach der Immobilisierung der sich drehenden Scheiben inkubiert man das inokulierte Milieu während der für die Reifung der Konidien erforderlichen Zeit, wobei man in dem Fermenter eine Zirkulation von Luft, Feuchtigkeit und eine kontrollierte Temperatur aufrechterhält, und

i) nach der gleichmäßigen Entwicklung der Sporen an der Oberfläche der Scheiben des Fermenters trennt man ab und gewinnt man die Sporen durch Abspülen der sich drehenden Scheiben mittels einer

EP 0 223 809 B1

schnellen Drehbewegung unter Verwendung einer Flüssigkeit, die vorzugsweise ein oberflächenaktives Agens und/oder kalibrierte Kugeln enthält, wobei die Mycel-Biomasse in dem erstarrten Kulturmedium eingeschlossen bleibt, das auf den Scheiben des Fermenters fixiert bleibt.

2. Verfahren nach Anspruch 1, dadurch gekennzeichnet, daß es angewendet wird zur Gewinnung von Sporen von Pilzen, deren Reproduktionsform aus Konidien besteht.

3. Verfahren nach einem der Ansprüche 1 und 2, dadurch gekennzeichnet, daß es angewendet wird auf die Gewinnung von Sporen von Pilzen, die zu den Genus Aspergillus, wie Aspergillus niger, Trichoderma, wie Trichoderma herzianum, oder Penicillium, wie Penicillium camembertii, gehören.

4. Verfahren nach einem der Ansprüche 1 bis 3, dadurch gekennzeichnet, daß die Kulturagentien bestehen aus einer Kohlenstoffquelle, wie Stärke, einer Stickstoffquelle, wie einem Gemisch aus Sulfat, Ammonium und Harnstoff, einer Phosphorquelle und anderen mineralischen oder organischen Verbindungen.

5. Verfahren nach einem der Ansprüche 1 bis 4, dadurch gekennzeichnet, daß es sich bei dem während der Stufe (a) zugegebenen Erstarrungsmittel um Agar-Agar handelt.

6. Verfahren nach einem der Ansprüche 1 bis 5, dadurch gekennzeichnet, daß die in der Stufe (i) verwendete Waschflüssigkeit steriles Wasser ist, dem ein oberflächenaktives Agens, wie TWEEN 80, zugesetzt worden ist.

7. Verfahren nach einem der Ansprüche 1 bis 6, dadurch gekennzeichnet, daß die in der Stufe (i) verwendeten kalibrierten Kugeln einen Durchmesser haben, der kleiner ist als der Abstand zwischen den Scheiben.

8. Verfahren nach einem der Ansprüche 1 bis 7, dadurch gekennzeichnet, daß die Suspension der abgetrennten Sporen durch Zentrifugieren oder Dekantieren konzentriert wird, gewaschen und danach getrocknet wird durch Eindampfen unter Vakuum, durch Lyophilisierung oder Versprühen.

9. Vorrichtung zur Durchführung des Verfahrens nach einem der Ansprüche 1 bis 8, dadurch gekennzeichnet, daß sie in Form eines Fermenters mit sich drehenden Scheiben vorliegt, der Systeme zur Regulierung der Temperatur und der Feuchtigkeit und einen Motor aufweist, der es ermöglicht, die Achse in Rotation zu versetzen, welche den Stapel von Scheiben trägt, von denen mindestens eine Oberfläche aus einem Sieb mit einer Maschenweite von 2 bis 5 mm besteht.

## Claims

1. Process for producing spores of fibrous mushrooms, characterized in that it involves the implementation in a rotary-disc fermenter of the following successive operations:

a) a solidifying agent is added to a culture medium containing at least one growing substrate and culturing agents;

b) this culture medium is placed on the rotary discs in the fermenter;

c) the whole of the fermenter is sterilized;

d) the culture medium is cooled to a temperature remaining above the solidifying point of the said medium;

e) the said culture medium is inoculated with spores of fibrous mushrooms;

f) the said culture medium is homogenized by rotating the discs of the fermenter slowly;

g) while maintaining a slow rotation of the fermenter discs, the medium is solidified by lowering the temperature of the fermenter abruptly, which has the effect of distributing the medium on the discs;

h) after bringing the rotary discs to a standstill, the inoculated medium is left to incubate while providing a circulation of air in the fermenter with controlled humidity and temperature during the time required for the conidia to mature, and

i) after the spores have developed uniformly on the surface of the fermenter discs, the spores are separated and harvested by sweeping the moving discs in a rapid rotary motion, with the aid of a fluid preferably containing a surfactant and/or sized balls, the mycelial biomass remaining imprisoned in the solidified culture medium which remains fixed on the fermenter discs.

2. Process according to Claim 1, characterized in that it is applied in the production of spores of mushrooms whereof the type of reproduction is by means of conidia.

3. Process according to one of Claims 1 and 2, characterized in that it is applied in the production of spores of mushrooms belonging to the species Aspergillus, such as Aspergillus niger, Trichoderma, such as Trichoderma harzianum, or Penicillium such as Penicillium camembertii.

4. Process according to one of Claims 1 to 3, characterized in that the culture agents are in the form of a carbon source such as starch, a nitrogen source such as a mixture of ammonium sulphate and urea, a phosphorous source and other mineral or organic compounds.

5. Process according to one of Claims 1 to 4, characterized in that the solidifying agent added in the course of step a) is agar-agar.

6. Process according to one of Claims 1 to 5, characterized in that the washing fluid used in the course of step i) is sterile water to which a surfactant such as TWEEN 80 has been added.

7. Process according to one of Claims 1 to 6, characterized in that the sized balls used in the course of step i) have a diameter less than the interface distance between the discs.

8. Process according to one of Claims 1 to 7, characterized in that suspension of harvested spores is

7

concentrated by centrifuging or decanting, washed and then dried by evaporation in vacuo, freeze-drying or atomization.

9. Device for implementing the process according to one of Claims 1 to 8, characterized in that it is in the form of a rotary-disc fermenter provided with systems for regulating the temperature and the humidity, and with a motor enabling the shaft supporting the stacked arrangement of discs to be rotated, at least one surface of these discs being formed by a screen having a mesh size from 2 to 5 mm.